# EUROPEAN PATENT APPLICATION

(11) **EP 1 431 756 A1**
(43) Date of publication of application: **23.06.2004**
(21) Application number: 02772942.5
(22) Date of filing: 27.09.2002
(51) Int. Cl.: G01N 27/12

(54) **SULFUR COMPONENT SENSOR AND SULFUR COMPONENT DETECTOR**

(30) Priority: 28.09.2001 JP 2001299036
(71) Applicant: SHINKO ELECTRIC INDUSTRIES CO. LTD., Nagano-shi, Nagano 380-0921 (JP)
(72) Inventor: HORIUCHI, Michio, SHINKO ELECTRIC IND. CO. LTD., Nagano-shi, Nagano 380-0921 (JP); SUGANUMA, Shigeaki, SHINKO ELECTRIC IND. CO. LTD., Nagano-shi, Nagano 380-0921 (JP); WATANABE, Misa, SHINKO ELECTRIC IND. CO. LTD., Nagano-shi, Nagano 380-0921 (JP)
(74) Representative: Patentanwälte Ruff, Wilhelm, Beier, Dauster & Partner
(86) International application number: PCT/JP2002/010106
(87) International publication number: WO 2003/029801

(57) **Abstract**

A sulfur-detecting sensor for detecting an integrated amount of hydrogen sulfide present in a gas stream passed over the sensor, characterized in that the sensor comprises a member formed of a material which is stable to hydrogen sulfide, and having a contacting side which is brought into contact with hydrogen sulfide, and a measuring member located on the contacting side of the former member, the measuring member being formed of a material which reacts with hydrogen sulfide to yield a reaction product having an increased electrical resistance value and/or making a phase change into a phase easily scattered by the gas stream at a temperature of the vicinity of the contacting side, and allowing an integrated amount of hydrogen sulfide passed over the sensor to be measured by measuring its electrical resistance value. A sulfur-detecting device using the detecting sensor is also disclosed.

## Description

### TECHNICAL FIELD

The invention relates to a sulfur-detecting sensor and a sulfur-detecting device. More particularly, the invention relates to a sulfur-detecting sensor and a sulfur-detecting device capable of measuring an accumulated amount of hydrogen sulfide (H₂S) present in a substantially oxygen-free atmosphere and having passed over the sensor or device.

### BACKGROUND ART

Recently, fuel cells have been studied in order to apply them to various uses such as home uses or automobile uses.

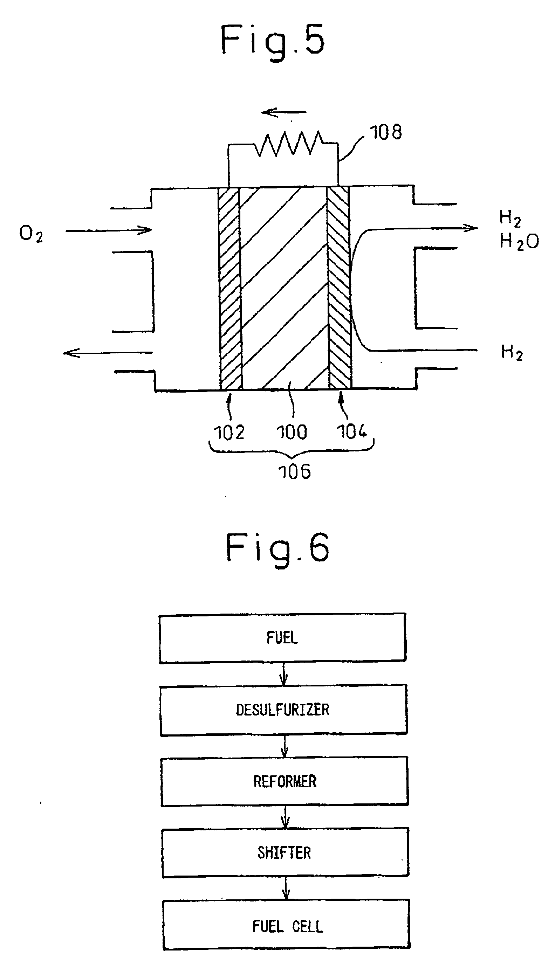

Such a fuel cell comprises a unit 106, for a fuel cell, made up of a solid electrolyte layer 100 of an oxygen ion-conductive type made of a sintered body of a stabilized zirconia to which yttria (Y₂O₃) is added, a cathode layer 102 formed on one side of the solid electrolyte layer 100, and an anode layer 104 formed on the other side of the solid electrolyte layer 100, as shown in Fig. 5. Oxygen or an oxygen-containing gas is fed to the side of the cathode layer 102 of the unit 106 for fuel cell. Hydrogen, as a fuel gas, is fed to the side of the anode layer 104. Development of polymeric fuel cells using a hydrogen ion conductor in place of the oxygen ion conductor have been promoted for the practical use thereof.

Oxygen (O₂) fed to the side of the cathode layer 102 of the unit 106, for a fuel cell, shown in Fig. 5 is ionized into oxygen ions (O²⁻) at the boundary between the cathode layer 102 and the solid electrolyte layer 100, and the oxygen ions (O²⁻) are conducted to the anode layer 104 through the solid electrolyte layer 100. The oxygen ion (O²⁻) conducted to the anode layer 104 reacts with hydrogen fed to the side of the anode layer 104 to form water (H₂O). During the reaction, the oxygen ions release electrons, so that a potential difference is generated between the cathode layer 102 and the anode layer 104. By electrically connecting the cathode layer 102 and the anode layer 104 to each other by a lead wire 108, the electrons of the anode layer 104 pass through the lead wire 108 toward the cathode layer 102 (in the direction of the arrow in the drawing), and electricity can be taken out from the fuel cell.

Hydrogen fed to the side of the anode layer 104 of the fuel cell of an oxygen ion conductive type, shown in Fig. 5, or hydrogen fed to the side of the anode layer of a fuel cell of a hydrogen ion conductive type are designed to be obtained, for domestic or automobile use, by the reformation of a fuel, such as gasoline or a commercial gas, which is easily available and is easily stored. Gasoline may contain a residual sulfur compound, such as thiophene, from a raw material, such as a crude oil, and a commercial gas has a sulfur compound, such as ethyl mercaptan, added to make it smell in order to allow a person to quickly discover the leakage of the gas.

The presence of such a sulfur compound reduces the activity of a reforming catalyst used for the reformation of a fuel, such as gasoline or a commercial gas, and, accordingly, a reforming process has a desulfurizer provided, as shown in the process block diagram of Fig. 6. The sulfur compound contained in a fuel, such as gasoline or a commercial gas is removed by feeding a gasified fuel to a desulfurizer. Subsequently, the fuel gas, from which the sulfur compound has been removed, is hydrogenated in a reformer, and CO contained in the gas is converted into CO₂ in a shifter before the hydrogenated gas is fed to a fuel cell.

In the reforming process shown in Fig. 6, when the desulfurizer functions normally, other equipment in the reforming process can also functions normally, to feed hydrogen to a fuel cell. However, when the adsorbing function of a desulfurization catalyst or the like filled in the desulfurizer is reduced, a poorly desulfurized fuel gas is fed to the reformer and the shifter, to thereby reduce the functions of the reformer and the shifter, and to further reduce the power generation capacity of the fuel cell. on this account, it is required that a measuring means, which can measure the sulfurous component in a fuel gas desulfurized in the desulfurizer, is installed between the desulfurizer and the reformer.

Such a measuring means must be capable of continuously measuring a sulfurous compound in a substantially oxygen-free gas stream. Although a measuring device for measuring a content of a sulfurous compound contained in a gas stream is used in a chemical plant or the like, this device is very large and is therefore impossible to use for domestic or automobile use.

Even if the fuel gas stream desulfurized in the desulfurizer has as small a content of sulfur compound as the functions of the reformer and the shifter are retained, the functions of the reformer and the shifter are gradually reduced, depending on an accumulated amount of sulfur compound fed thereto, when the fuel gas containing the sulfur compound is continually fed. On this account, it is also needed to measure an accumulated amount of sulfur compound fed to the reformer and the shifter. However, the devices for measuring a sulfur compound in a gas stream used in traditional chemical plants cannot measure an accumulated amount of sulfur compound in a gas stream passed over a device.

Thus, an object of the invention is to provide a sulfur-detecting sensor and a sulfur-detecting device which are compact so as to be capable of being used for domestic or automobile use and can measure an accumulated amount of a sulfur compound passed over the sensor or device.

### DISCLOSURE OF THE INVENTION

To solve the above problems, the inventors first investigated sulfur compounds present in a stream of substantially oxygen-free gas, such a fuel gas desulfurized in a desulfurizer, and have discovered that they are mostly hydrogen sulfide (H₂S). They have further discovered that a reaction product formed by the reaction of a certain metal with hydrogen sulfide (H₂S) has a high electrical resistance value or a low melting point compared to the metal before the reaction, or exhibits the nature of sublimation.

The inventors have further investigated, based on the above knowledge, and, consequently, have found that, by placing, in a gas stream containing H₂S, a member of a wire or the like formed of a material which reacts with H₂S to yield a reaction product having an increased electrical resistance value, or to yield a reaction product making a phase change into a phase easily to be scattered by a gas stream, and measuring the electrical resistance value of the member, an accumulated amount of H₂S having passed over the member can be measured, and have conceived of the invention.

The invention resides in a sulfur-detecting sensor for detecting an integrated amount of hydrogen sulfide (H₂S) present in a gas stream having passed over the sensor, characterized in that the sensor comprises a member formed of a material which is stable to hydrogen sulfide, and having a contacting side which is brought into contact with hydrogen sulfide, and a measuring member located on the contacting side of the former member, the measuring member being formed of a material which reacts with hydrogen sulfide to yield a reaction product having an increased electrical resistance value and/or making a phase change into a phase easily scattered by the gas stream at a temperature of the vicinity of the contacting side, and allowing an integrated amount of hydrogen sulfide having passed over the sensor to be measured by measuring its electrical resistance value.

The invention also resides in a sulfur-detecting device for detecting an integrated amount of hydrogen sulfide (H₂S) present in a gas stream having passed over the device, characterized in that there is provided a sulfur-detecting sensor comprising a member formed of a material which is stable to hydrogen sulfide, and having a contacting side which is brought into contact with hydrogen sulfide, and a measuring member located on the contacting side of the former member, the measuring member being formed of a material which reacts with hydrogen sulfide to yield a reaction product having an increased electrical resistance value and/or making a phase change into a phase easily scattered by the gas stream at a temperature of the vicinity of the contacting side, and allowing an integrated amount of hydrogen sulfide passed over the sensor to be measured by measuring its electrical resistance value, and a means for measuring the electrical resistance value of the measuring member of the sensor.

The measuring member in the sulfur-detecting sensor of the invention may be a pattern formed on the contacting side brought into contact with hydrogen sulfide, or a wire arranged on the contacting side.

It is preferred that such a measuring member is formed of zinc (Zn), silver (Ag), tin (Sn), iron (Fe), nickel (Ni), molybdenum (Mo) or copper (Cu). When these metal materials react with hydrogen sulfide, they form a reaction product having an increased electrical resistance value and/or making a phase change into a phase easily scattered by a gas stream at a temperature of the vicinity of the contacting side. On this account, the measuring member made of the material reacts with hydrogen sulfide, to thereby be changed to a reaction product having a high electrical resistance value, and/or to a reaction product making a phase change into a phase easily scattered by a gas stream at a temperature of the vicinity of the contacting side, and to gradually become thinner, resulting in the increased electrical resistance value of the material. Therefore, by measuring the amount of change in electrical resistance value of the measuring member after a lapse of a predetermined time, the accumulated amount of hydrogen sulfide in the gas stream passed over the sensor within the predetermined time can be measured.

As the measuring member is formed as a pattern or a wire of a specific metal, the sulfur-detecting sensor of the invention can be formed very compactly, and can be satisfactorily used for domestic or automobile applications.

In addition, the sulfur-detecting sensor can be suitable used for the measurement of hydrogen sulfide (H₂S) in a substantially oxygen-free gas stream, unlike conventional sulfur-detecting sensors which carry out the measurement in the presence of oxygen.

### BRIEF DESCRIPTION OF THE DRAWINGS

Fig. 1 is an illustration of an exemplification of the sulfur-detecting device of the invention.
Fig. 2 is a perspective view of the sulfur-detecting sensor used in the sulfur-detecting device shown in Fig. 1.
Fig. 3 is a perspective view of illustrating another exemplification of the sulfur-detecting device of the invention.
Fig. 4 is an illustration of the another exemplification of the sulfur-detecting device of the invention.
Fig. 5 a schematic illustration of a fuel cell in which the sulfur-detecting device of the invention is used.
Fig. 6 is a block diagram illustrating a process for reforming a fuel gas fed to the fuel cell shown in Fig. 5.
Fig. 7 shows the change in resistance of the copper wire of Example 3 in a gas containing hydrogen sulfide.
Fig. 8 shows the change in resistance of the sample of Example 4 in a gas containing hydrogen sulfide.
Fig. 9 shows the change in resistance of the sample of Example 5 in a gas containing hydrogen sulfide.
Fig. 10 shows the change in resistance of the sample of Example 5 in a hydrogen sulfide-free gas.
Fig. 11 shows the change in resistance of the sample of Example 6 in a gas containing hydrogen sulfide.

### BEST MODE FOR CARRYING OUT THE INVENTION

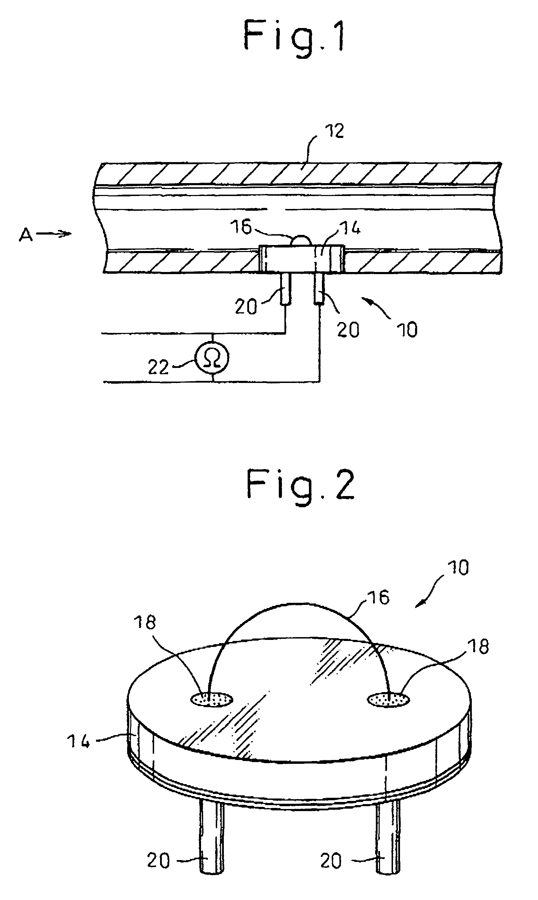

Fig. 1 shows an exemplification of the sulfur-detecting device of the invention. A sulfur-detecting sensor 10 used in the sulfur-detecting device shown in Fig. 1 is provided at a pipe through which a gas containing hydrogen sulfide (H₂S) flows in the direction of the arrow A.

As shown in Fig. 2, the sulfur-detecting sensor 10 is provided, at one side (contacting side) of a substrate 14 formed of an insulating material, such as a ceramic, which is stable to hydrogen sulfide, with a wire 16 so that it is brought into contact with the gas containing hydrogen sulfide (H₂S) passing through the inside of the pipe 12. Each of the ends of the wire 16 is inserted in a through-hole 18 formed in the substrate 14, is sealed, and is electrically connected to a lead 20 provided at the other side of the substrate 14.

The wire 16 is formed of a material which reacts with hydrogen sulfide to yield a reaction product having an increased electrical resistance value and/or making a phase change into a phase easily scattered by a gas stream at a temperature of the vicinity of the wire 16. The "phase change" herein means that a reaction product formed by a reaction with hydrogen sulfide is changed into a state in which the product is easily scattered by a gas stream flowing through the inside of the pipe 12, by, for example, melting, sublimation, pyrolysis or the like at a temperature of the vicinity of the wire 16. For example, in the case where the reaction product melts (changes into a liquid phase), the melt is entrained with a gas stream to be scattered, or is moved by flowing, resulting in the separation from the wire 16, and in the case of sublimation (change into a vapor phase), the reaction product is scattered into the gas stream as vapor.

As the materials forming such a reaction product, zinc (Zn), silver (Ag), tin (Sn), iron (Fe), nickel (Ni), molybdenum (Mo), and copper (Cu) can be enumerated.

With zinc (Zn), silver (Ag), tin (Sn), iron (Fe), nickel (Ni) or molybdenum (Mo) of these metal materials, the sulfide thereof has a higher electrical resistance value than that of the metal before the sulfurization. Particularly, sulfides (FeS, FeS₂, NiS, Ni₃S₄, Ni₃S₂, etc.) of iron (Fe) and nickel (Ni) have a melting point which is much lower than the melting point of iron (Fe) or nickel (Ni). On this account, the wire 16 formed of iron (Fe) or nickel (Ni) makes it possible to measure an accumulated amount of hydrogen sulfide having passed over the wire 16 in a short period of time, through the synergy of the increase in electrical resistance value due to a sulfide of a product of the reaction with hydrogen sulfide and the increase in electrical resistance value due to the decrease in thickness of the wire 16.

Although sulfides of copper (Cu) exhibit electrical resistance values similar to that of the metallic copper, the microstructures thereof become extremely open-packed. On this account, a sulfide of copper (Cu), which forms the wire 16, is scattered into the gas stream flowing the inside of the pipe 12, and the wire 16 is made thinner, resulting in an increase in the electrical resistance value of the wire 16.

With zinc (zn) and tin (Sn), the metals themselves have a low melting point. Therefore, the wire 16 made of zinc (Zn) or tin (Sn) is used in the case where the temperature of the contacting side of the substrate 14 is lower than the melting point of zinc (Zn) or tin (Sn).

As shown in Fig. 1, an electrical resistance measuring instrument 22, as a means for measuring the electrical resistance value of the wire 16, is connected to the leads 20 of the sulfur-detecting sensor 10 shown in Fig. 2. The electrical resistance measuring instrument 22 measures the electrical resistance value of the wire 16 by passing a given direct current through the wire 16. The measurement of the electrical resistance value of the wire 16 may be continuously or intermittently carried out. By comparing the electrical resistance value of the wire 16 thus measured with an electrical resistance value thereof measured a given time before, the accumulated amount of hydrogen sulfide having passed within the given time can be determined. In addition, the electrical resistance value of the wire 16 measured by the electrical resistance measuring instrument 22 may be converted to an amount of hydrogen sulfide for indication, or may be used so as to raise an alarm when the electrical resistance value of the wire 16 reaches a predetermined value to indicate that a predetermined amount of hydrogen sulfide has passed.

Having described the wire 16 made only of a certain metal, a wire of a core-sheath structure made of different materials for the core and sheath sections can also be used. Wires of such a core-sheath structure may include those in which the core and sheath sections are made of different types of metals, and those in which a metal layer, as the sheath section, of a certain metal material is formed around an electrically insulative ceramic fiber, as the core section.

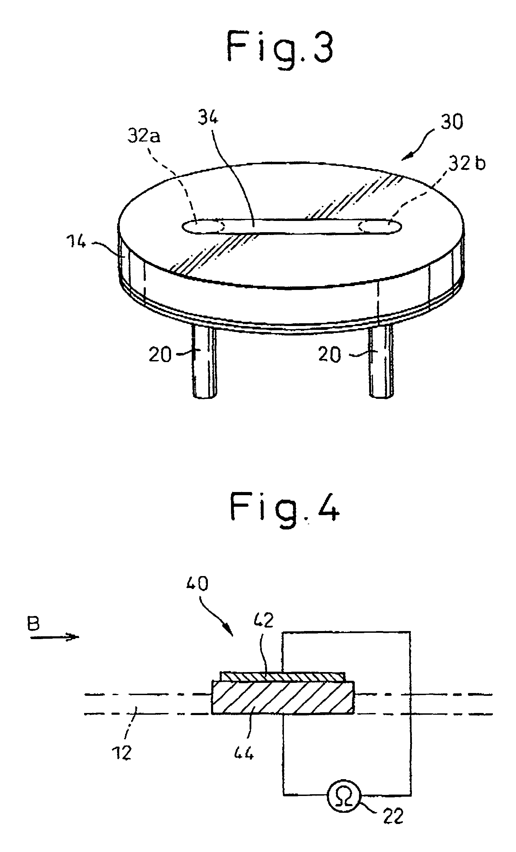

In lieu of the sulfur-detecting sensor 10 shown in Figs. 1 and 2, a sulfur-detecting sensor 30 shown in Fig. 3 can also be used. In the sulfur-detecting sensor 30, a pattern 34 is formed, which electrically connects two spots, for example, a pad 32a at the left and a pad 32b at the right, formed on one side, i.e., the contacting side brought into contact with a gas containing hydrogen sulfide (H₂S), of a substrate 14 made of an insulating material stable to hydrogen sulfide, such as a ceramic. Each of the pads 32a, 32b is electrically connected to a lead 20 provided at the other side of the substrate 14.

The pattern 34 shown in Fig. 3 may be formed by metallization with a prescribed metal, or by plating a pattern formed by metallization with a metal, such as tungsten, with a prescribed metal. The metal used for the formation of the pattern 34 can be selected from zinc (Zn), silver (Ag), tin (Sn), iron (Fe), nickel (Ni), molybdenum (Mo) and copper (Cu), as referred to above.

The electrical resistance value of the pattern 34 depends on the cross sectional area of the pattern 34. On this account, it is preferred that the pattern 34 is formed so as to have as even a thickness and width as possible.

The sulfur-detecting sensor 30 shown in Fig. 3, which uses the pattern 34 formed of a metal material, can also be installed in a pipe 12 through which a gas containing hydrogen sulfide is passed to measure the electrical resistance value of the pattern 34 by an electrical resistance measuring instrument 22, as shown in Fig. 1, to thereby measure an amount of hydrogen sulfide passed within a predetermined time or an accumulated amount of hydrogen sulfide passed after installing the sulfur-detecting sensor 30 to the pipe 12.

In the sulfur-detecting sensors 10, 30 shown in Figs 1 to 3, the wire 16 or pattern 34 positioned on the substrate 14 made of a ceramic of an insulating material is used as a measuring member. On this account, if the wire 16 has an uneven thickness, or the pattern 34 is not uniformly formed and has unevenness or the like at its surface, the wire 16 or pattern 34 may be broken at a portion thereof which is thinner than the remainder with the progress of a reaction of the wire 16 or pattern 34 with hydrogen sulfide. When the break occurs, there is a possibility that a measured value of the accumulated amount of hydrogen sulfide having passed over the sulfur-detecting sensor 10, 30 is extremely inaccurate.

In a sulfur-detecting sensor 40 shown in Fig. 4, a pattern 42 is formed over substantially the whole area of one side, i.e., the side brought into contact with hydrogen sulfide, of a substrate 44 made of a conductive material stable to hydrogen sulfide, and a total of the electrical resistance values of the whole area pattern 42 and the conductive substrate 44 is measured by an electrical resistance measuring instrument 22. Even if metal material is lost at a portion of the whole area pattern 42 by a reaction with hydrogen sulfide, the whole area pattern is not totally broken, and a possibility of inaccurate measured value of the accumulated amount of hydrogen sulfide can be eliminated.

The substrate 44 used in the sulfur-detecting sensor 40 shown in Fig. 4 forms part of the pipe 12, so that it must be gas-tight and stable to hydrogen sulfide, and exhibit electrical conductivity. As a material satisfying such conditions, a stainless steel or lanthanum strontium manganese oxide can be preferably used.

The sulfur-detecting sensors 10, 30, 40 of the invention shown in Figs. 2 to 4 can be downsized to the extent that they can be installed to the pipe 12. Consequently, the sulfur-detecting sensor of the invention can be installed to a pipe in a reforming process, which feeds a hydrogen-containing gas to a fuel cell for domestic or automobile use, as shown in Fig. 6. Particularly, by a sulfur-detecting device in which an electrical resistance measuring instrument connected to leads of a sulfur-detecting sensor provided at a pipe between a desulfurizer and a reformer, it can be determined whether or not the desulfurizer displays its desulfurization function satisfactorily. In the case where the desulfurization function of the desulfurizer is lowered, and a situation of the discharge of a hydrogen sulfide-containing fuel gas from the desulfurizer is invited, it can be detected by the installed sulfur-detecting device, and a response, such as an exchange of the desulfurizer, can be made. Thus, a reformer, a shifter and the like can be protected from a sulfur compound, and the improvement of the life of a fuel cell can be expected.

The invention will be further described by means of examples.

### Example 1

A strip-like pattern was formed on a circular ceramic substrate by rectangularly applying thereto a copper paste, and firing it. The electrical resistance value of the strip-like pattern measured 0.1 ohm. Subsequently, the ceramic substrate on which the strip-like pattern was formed was left to stand in a nitrogen gas stream containing 50 ppm of hydrogen sulfide at a flow rate of 500 milliliters per minute for 48 hours, while maintained at a temperature of 600°C. when the ceramic substrate was removed form the stream, and the strip-like pattern was observed with naked eyes, it had been discolored and was thinner. The electrical resistance value of the discolored and thinned strip-like pattern could not be measured.

### Example 2

A strip-like pattern was formed on a circular ceramic substrate by rectangularly applying thereto a tungsten paste, firing it, and then electroplating the rectangular pattern with nickel. The electrical resistance value of the strip-like pattern measured 0.5 to 0.6 ohm. Subsequently, the ceramic substrate on which the strip-like pattern was formed was left to stand in a nitrogen gas stream containing 50 ppm of hydrogen sulfide at a flow rate of 500 milliliters per minute for 48 hours, while maintained at a temperature of 600°C. When the ceramic substrate was removed form the stream, and the strip-like pattern was observed with naked eyes, it had been discolored. The discolored strip-like pattern had an electrical resistance value of 5 to 6 ohms, which was larger than the electrical resistance value at the formation of the strip-like pattern.

### Example 3

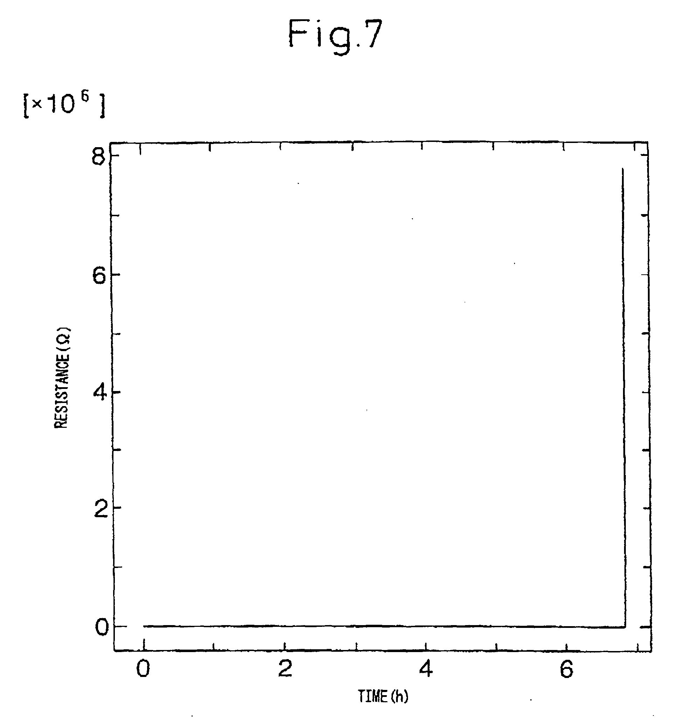

A platinum wire having a diameter of 0.4 millimeter was spot-welded to either end of a copper wire having a diameter of 0.4 millimeter and a length of about 5 millimeters, and the portion of platinum wire was inserted into a sheath made of zirconia ceramic. The resistance between the ends of the copper wire measured 1.76 ohms in a nitrogen gas stream at 600°C. The portion of the copper wire was exposed to a nitrogen gas stream containing 50 ppm of hydrogen sulfide at a flow rate of 500 millimeter per minute at 600°C and, as a consequence, the resistance increased to the order of 10⁶ ohms after a lapse of 6 hours, as shown in Fig. 7.

### Example 4.

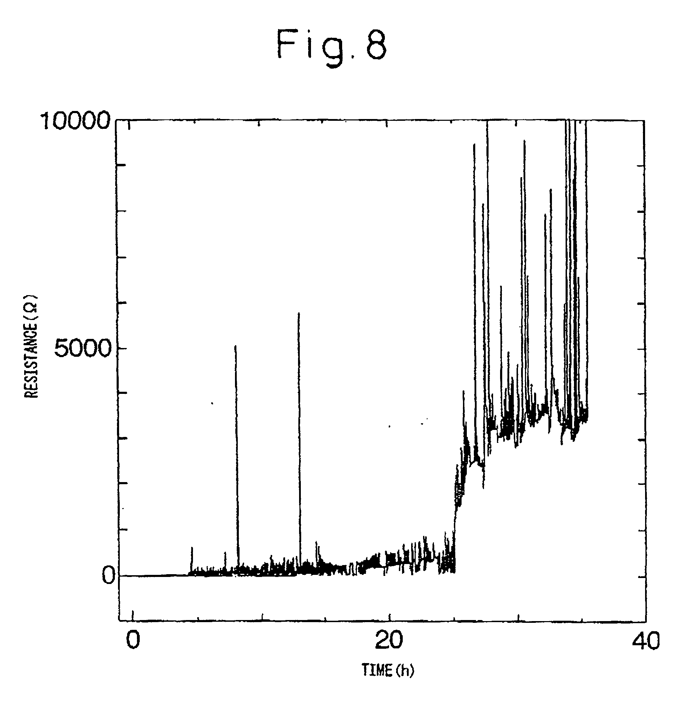

A sample was made by wedge-bonding a copper wire having a diameter of 20 micrometers between two terminals of a three-terminal TO header plated with gold. The resistance between the terminals measured at room temperature was not greater than 1 ohm. When the portion of the copper wire was exposed to a nitrogen gas stream containing 1 ppm of hydrogen sulfide at a flow rate of 500 millimeter per minute at 600°C, the resistance became unstable several hours later, as shown in Fig. 8, and the resistance value was increased to the order of 10⁵ ohms after 20 hours and a further unstable increase in resistance was observed. For comparison, when the same sample was exposed to a nitrogen gas stream containing no hydrogen sulfide at 500 millimeters per minute and 600°C, the resistance was stable, and an increase in resistance value was not observed for at least 130 hours or more.

### Example 5

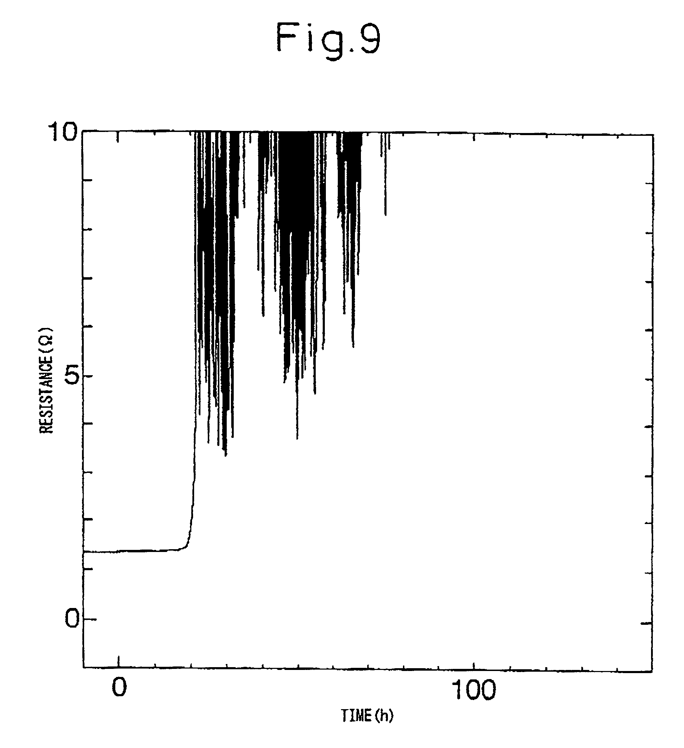

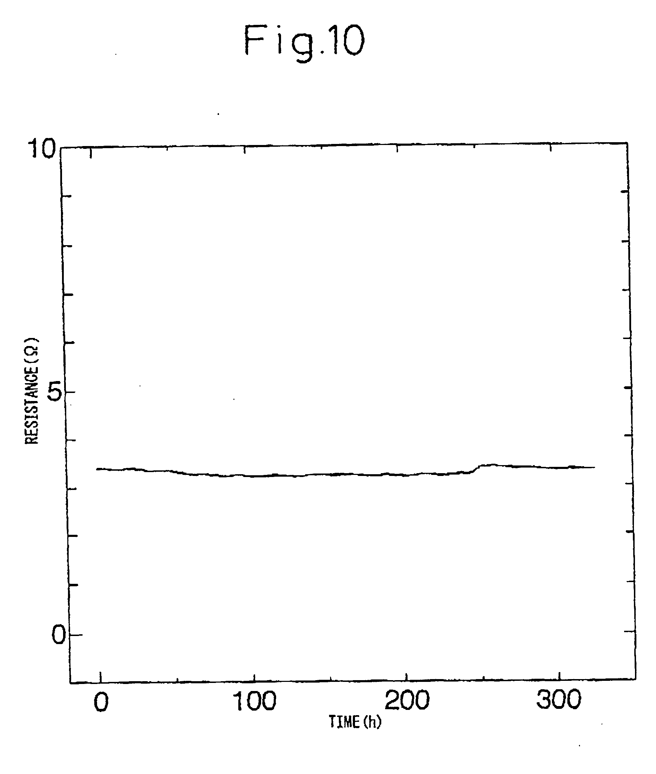

The same sample as in Example 4 was exposed to a nitrogen gas stream containing 0.5 ppm of hydrogen sulfide at 500 milliliters per minute and 200°C and, as a consequence, an abrupt increase in resistance was observed after 20 hours, as shown in Fig. 9. For comparison, when the same sample was exposed to a nitrogen gas stream containing no hydrogen sulfide at 500 millimeters per minute and 200°C, the resistance was stable, and an increase in resistance value was not observed for at least 300 hours or more, as shown in Fig. 10.

### Example 6

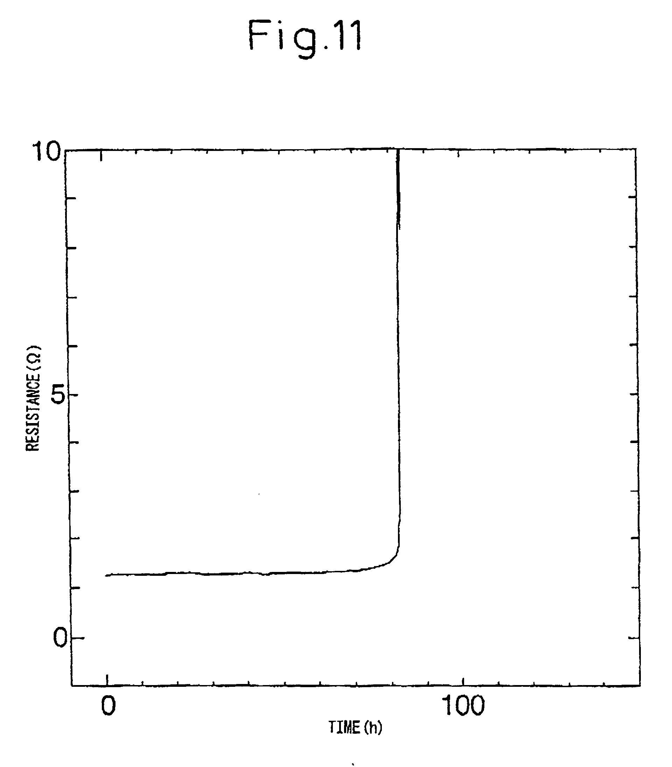

The same sample as in Example 4 was exposed to a nitrogen gas stream containing 0.1 ppm of hydrogen sulfide at 500 milliliters per minute and 200°C and, as a consequence, an abrupt increase in resistance was observed after 80 hours, as shown in Fig. 11.

### INDUSTRIAL APPLICABILITY

According to the sulfur-detecting sensor of the invention, an accumulated amount of hydrogen sulfide contained in a gas having passed within a predetermined time can be measured. The sulfur-detecting sensor of the invention can be downsized to the extent that it can be installed to a pipe through which a gas stream is passed, so that a sulfur-detecting device using it can be easily used as a device for domestic or automobile use.

## Claims

1. A sulfur-detecting sensor for detecting an integrated amount of hydrogen sulfide present in a gas stream having passed over the sensor, **characterized in that** the sensor comprises a member formed of a material which is stable to hydrogen sulfide, and having a contacting side which is brought into contact with hydrogen sulfide, and a measuring member located on the contacting side of the former member, the measuring member being formed of a material which reacts with hydrogen sulfide to yield a reaction product having an increased electrical resistance value and/or making a phase change into a phase easily scattered by the gas stream at a temperature of the vicinity of the contacting side, and allowing an integrated amount of hydrogen sulfide having passed over the sensor to be measured by measuring its electrical resistance value.

2. The sulfur-detecting sensor of claim 1, wherein the measuring member is a pattern formed on the contacting side or a wire arranged on the contacting side.

3. The sulfur-detecting sensor of claim 1, wherein the measuring member is a pattern formed on an underlayer of a material different from the material of the measuring member, the underlayer being formed on the contacting side of the member having a contacting side which is brought into contact with hydrogen sulfide.

4. The sulfur-detecting sensor of claim 1, wherein the measuring member is a pattern formed over substantially the whole area of the contacting side of the member having a contacting side which is brought into contact with hydrogen sulfide.

5. The sulfur-detecting sensor of claim 1, wherein the measuring member is a sheath section of a core-sheath structure in which a core section and a sheath section are formed of different materials.

6. The sulfur-detecting sensor of any one of claims 1 to 5, wherein the measuring member is made of zinc, silver, tin, iron, nickel, molybdenum or copper.

7. The sulfur-detecting sensor of any one of claims 1 to 6, wherein the gas stream is a substantially oxygen-free gas stream.

8. A sulfur-detecting device for detecting an integrated amount of hydrogen sulfide (H₂S) present in a gas stream having passed over the device, **characterized in that** there is provided a sulfur-detecting sensor comprising a member formed of a material which is stable to hydrogen sulfide, and having a contacting side which is brought into contact with hydrogen sulfide, and a measuring member located on the contacting side of the former member, the measuring member being formed of a material which reacts with hydrogen sulfide to yield a reaction product having an increased electrical resistance value and/or making a phase change into a phase easily scattered by the gas stream at a temperature of the vicinity of the contacting side, and allowing an integrated amount of hydrogen sulfide having passed over the sensor to be measured by measuring its electrical resistance value, and a means for measuring the electrical resistance value of the measuring member of the sensor.

9. The sulfur-detecting device of claim 8, wherein the measuring member of the sulfur-detecting sensor is a pattern formed on the contacting side or a wire arranged on the contacting side.

10. The sulfur-detecting device of claim 8, wherein the measuring member of the sulfur-detecting sensor is a pattern formed on an underlayer of a material different from the material of the measuring member, the underlayer being formed on the contacting side of the member having a contacting side which is brought into contact with hydrogen sulfide.

11. The sulfur-detecting device of claim 8, wherein the measuring member of the sulfur-detecting sensor is a pattern formed over substantially the whole area of the contacting side of the member having a contacting side which is brought into contact with hydrogen sulfide.

12. The sulfur-detecting device of claim 8, wherein the measuring member of the sulfur-detecting sensor is a sheath section of a core-sheath structure in which a core section and a sheath section are formed of different materials.

13. The sulfur-detecting device of any one of claims 8 to 12, wherein the measuring member of the sulfur-detecting sensor is made of zinc, silver, tin, iron, nickel, molybdenum or copper.

14. The sulfur-detecting device of any one of claims 8 to 13, wherein the gas stream is a substantially oxygen-free gas stream.
